(19) Europäisches Patentamt / European Patent Office / Office européen des brevets

(11) **EP 3 649 252 B1**

(12) **EUROPÄISCHE PATENTSCHRIFT**

(45) Veröffentlichungstag und Bekanntmachung des Hinweises auf die Patenterteilung:
**07.09.2022  Patentblatt 2022/36**

(21) Anmeldenummer: **18727785.0**

(22) Anmeldetag: **28.05.2018**

(51) Internationale Patentklassifikation (IPC):
*C12Q 1/25* (2006.01)    *B01L 3/00* (2006.01)
*G01N 33/18* (2006.01)    *G01N 33/49* (2006.01)

(52) Gemeinsame Patentklassifikation (CPC):
**C12Q 1/25; G01N 33/18; G01N 33/49;** B01L 3/5027

(86) Internationale Anmeldenummer:
**PCT/EP2018/063865**

(87) Internationale Veröffentlichungsnummer:
**WO 2019/007588 (10.01.2019 Gazette 2019/02)**

(54) **VORRICHTUNG UND VERFAHREN ZUM NACHWEIS EINES BESTIMMTEN ANALYTEN IN EINER FLÜSSIGEN PROBE UND VERWENDUNGEN DER VORRICHTUNG**

DEVICE AND METHOD FOR DETECTING A SPECIFIC ANALYTE IN A LIQUID SAMPLE AND USES OF SAID DEVICE

DISPOSITIF ET PROCÉDÉ DE MISE EN ÉVIDENCE D'UN ANALYTE DÉTERMINÉ DANS UN ÉCHANTILLON LIQUIDE ET UTILISATIONS DU DISPOSITIF

(84) Benannte Vertragsstaaten:
**AL AT BE BG CH CY CZ DK EE ES FI FR GB GR HR HU IE IS IT LI LT LU LV MC MK MT NL NO PL PT RO RS SE SI SK SM TR**

(30) Priorität: **05.07.2017   DE 102017211478**

(43) Veröffentlichungstag der Anmeldung:
**13.05.2020   Patentblatt 2020/20**

(73) Patentinhaber: **Anvajo GmbH**
**01069 Dresden (DE)**

(72) Erfinder: **FRAEDRICH, Stefan**
**01744 Dippoldiswalde (DE)**

(74) Vertreter: **Pfenning, Meinig & Partner mbB**
**Patent- und Rechtsanwälte**
**An der Frauenkirche 20**
**01067 Dresden (DE)**

(56) Entgegenhaltungen:
**EP-A2- 0 663 239      EP-A2- 0 803 731**
**WO-A1-2009/125227   JP-A- S5 788 358**
**JP-A- S6 052 765**

**Beschreibung**

Erfindungsgemäß wird eine Vorrichtung und ein Verfahren zum Nachweis eines bestimmten Analyten in einer flüssigen Probe bereitgestellt. Die Vorrichtung, die in dem Verfahren verwendet werden kann, enthält mindestens eine Fluidleitung, mindestens einen Aufnahmebereich zur Aufnahme einer flüssigen Probe, mindestens einen Enzymbereich mit mindestens einem bestimmten Enzym und/oder mindestens einen Ansäuerungsbereich mit mindestens einer Säure. Ferner enthält die Vorrichtung mindestens einen Reaktionsbereich, der zur Ausbildung von Gasbläschen geeignet ist. Die mindestens eine Fluidleitung ist dazu geeignet, die flüssige Probe - über Kapillarkräfte und/oder mindestens eine Mikropumpe in der Fluidleitung - vom Aufnahmebereich über den Enzymbereich und/oder den Ansäuerungsbereich zum Reaktionsbereich zu transportieren. Die Vorrichtung ermöglicht einen schnellen, einfachen und kostengünstigen Nachweis eines bestimmten Analyten in einer flüssigen Probe, wobei der Nachweis mit einer hohen Detektionssensitivität, Detektionsspezifität und Detektionspräzision möglich ist. Ferner werden Verwendungen der erfindungsgemäßen Vorrichtung vorgeschlagen.

Biomarker sind messbare Parameter für biologische Prozesse, die Rückschlüsse auf Erkrankungen und den physiologischen Zustand zulassen. Zu den bekanntesten Biomarker-Molekülen gehören vor allem Hormone (z.B. T3/T4) Stoffwechselprodukte, Blutzucker als auch Cholesterin und andere Blutfette.

Biomarker liegen oft in sehr geringen Konzentrationen und im Komplex mit einer Vielzahl anderer Proteine vor, was die Charakterisierung sowohl erschwert als auch die Analyse kosten- und zeitaufwendig gestaltet. Kommerziell erhältliche, freiverkäufliche Schnelltests basieren meist auf einer qualitativen ja/nein-Antwort, aber erlauben keine quantitative Beurteilung der Konzentration eines bestimmten Analyten in einer flüssigen Probe.

Zum Nachweis von Biomarkern als Analyten werden sehr häufig serologische Methoden angewandt. Die Nachteile dieser Methoden sind ein hoher experimenteller Aufwand, da einerseits eine Auswahl geeigneter Antikörper getroffen werden muss, die Antikörper markiert werden müssen (z.B. mit einem Fluoreszenzfarbstoff) und die Bestimmungsverfahren zeitaufwändig sind. Ferner sind diese Bestimmungsverfahren mit einer hohen Detektionsunschärfe behaftet.

Viele der im Stand der Technik bekannten Verfahren zum qualitativen bzw. quantitativen Nachweis eines bestimmten Analyten in einer flüssigen Probe basieren auf der Detektion hochspezifischer Bindungsereignisse zwischen einem Epitop des zu bestimmenden Analyten (z.B. ein Epitop eines Antigens) und einem bestimmten Paratop am Detektormolekül (z.B. einem Paratop eines Antikörpers). Bekannte Verfahren zur Identifikation von Biomarkern sind beispielsweise der "enzyme-linked immunosorbant assay" ("ELISA"), die Gelelektrophorese, die Oberflächenplasmonenresonanzspektroskopie (SPR-Spektroskopie), die Verwendung von "protein microarrays" (z.B. "masssensing BioCD protein array"), die oberflächenverstärkte Raman-Spektroskopie, kolorimetrische oder elektrochemische Verfahren und die Fluoreszenzspektroskopie.

Aktueller Goldstandard in der Diagnostik von Biomarkern sind Immunoassays, bei denen ein Fängerantikörper an eine feste Phase immobilisiert ist und die Reaktion mit dem Antigen mithilfe eines Sekundärantikörpers ausgelesen wird. Nachteil an diesen Immunoassays ist, dass unspezifische Bindungen von anderen Proteinen an die Sensoroberfläche das Ergebnis falsch positiv beeinflussen und damit stark verfälschen können.

WO2009/125227 offenbart Vorrichtungen und Methoden zum Nachweis von Analyten durch Gasbläschenbildung mittels isolierter Enzyme oder flüssiger Säuren.

Insgesamt sind die gegenwärtigen Verfahren zur Identifikation von Biomarkern durch einen hohen Zeitaufwand, einen hohen apparativen Aufwand, einen Bedarf an Fachpersonal, eine geringe Detektionssensitivität, eine geringe Detektionsspezifität und eine geringe Detektionsvarianz gekennzeichnet. Ausgehend hiervon war es die Aufgabe der vorliegenden Erfindung, eine Vorrichtung und ein Verfahren bereitzustellen, die/das einen schnellen, einfachen und kostengünstigen Nachweis eines bestimmten Analyten in einer flüssigen Probe ermöglicht, der/das zudem eine hohe Detektionssensitivität, eine hohe Detektionsspezifität und eine hohe Präzision in der Detektion ermöglichen. Ferner sollten Verwendungen einer solchen Vorrichtung vorgeschlagen werden.

Die Aufgabe wird gelöst durch die Vorrichtung mit den Merkmalen von Anspruch 1, dem Verfahren mit den Merkmalen von Anspruch 14 und der Verwendung mit den Merkmalen von Anspruch 18. Die abhängigen Ansprüche zeigen vorteilhafte Weiterbildungen auf.

Erfindungsgemäß wird eine Vorrichtung zum Nachweis eines bestimmten Analyten in einer flüssigen Probe über enzymkatalysierte und/oder säurevermittelte Umsetzung des Analyten zu mindestens einem Gas bereitgestellt, enthaltend

    a) mindestens eine Fluidleitung;
    b) mindestens einen Aufnahmebereich zur Aufnahme einer flüssigen Probe;
    c) i) mindestens einen Enzymbereich, der mindestens ein bestimmtes Enzym enthält, das dazu geeignet ist, die Umsetzung des zu bestimmenden

Analyten zu mindestens einem Gas zu katalysieren, wobei der mindestens eine Enzymbereich biologische Zellen und/oder Zelllysat enthält, wobei die biologischen Zellen und/oder das Zelllysat das mindestens eine Enzym enthalten, das dazu geeignet ist, die Umsetzung des zu bestimmenden Analyten zu mindestens einem Gas zu katalysieren; und/oder

ii) mindestens einen Ansäuerungsbereich, der mindestens eine Säure enthält, die ausgewählt ist aus der Gruppe bestehend aus bei Raumtemperatur und Normaldruck festen Säuren; und d) mindestens einen Reaktionsbereich zur Ausbildung von Gasbläschen, wobei der Reaktionsbereich eine Kammer enthält oder daraus besteht, die mit der mindestens einen Fluidleitung fluidisch verbunden ist und flüssigkeitsdichte Wandungen aufweist; dadurch gekennzeichnet, dass die mindestens eine Fluidleitung dazu geeignet ist, über Kapillarkräfte und/oder mindestens eine Mikropumpe in der Fluidleitung, die flüssige Probe vom Aufnahmebereich über den mindestens einen Enzymbereich und/oder den mindestens einen Ansäuerungsbereich zum Reaktionsbereich zu transportieren.

Die erfindungsgemäße Vorrichtung kann somit entweder mindestens einen Enzymbereich mit den oben genannten Merkmalen oder mindestens einen Ansäuerungsbereich mit den oben genannten Merkmalen aufweisen oder aber sowohl mindestens einen Enzymbereich mit den oben genannten Merkmalen als auch mindestens einen Ansäuerungsbereich mit den oben genannten Merkmalen aufweisen.

Die Vorrichtung weist eine Mikropumpe auf, die dazu geeignet ist, die flüssige Probe aktiv (d.h. über einen Energieeintrag) vom Aufnahmebereich über den mindestens einen Enzymbereich und/oder den mindestens einen Ansäuerungsbereich zum Reaktionsbereich zu transportieren. Dieser aktive Transport kann einen passiven Transport (d. h. einen Transport über Kapillarkräfte) unterstützen. Die Vorrichtung (insbesondere die mindestens eine Fluidleitung) ist alternativ auch ohne Mikropumpe dazu geeignet, die flüssige Probe passiv (d.h allein über Kapillarkräfte) vom Aufnahmebereich über den mindestens einen Enzymbereich und/oder den mindestens einen Ansäuerungsbereich zum Reaktionsbereich zu transportieren.

Im Falle des Transports über mindestens eine Mikropumpe weist die erfindungsgemäße Vorrichtung bevorzugt eine Energiequelle auf, die bevorzugt ausgewählt ist aus Batterie, Akkumulator, photovoltaisches Element und Kombinationen hiervon. Die für den Betrieb der Mikropumpe nötige Energiequelle kann jedoch auch in einem optischen Detektionsgerät enthalten sein und die Vorrichtung bei Anordnung der Vorrichtung in oder an dem optischen Detektionsgerät die Energie durch die Energiequelle beziehen.

Die Spezifikation bezieht sich auf eine Vorrichtung zur Detektion von Biomarkern mithilfe gaserzeugende chemischer Reaktionen in einer geometrisch begrenzten Probenkammer. Durch diese Vorrichtung können bestimmte Analyten (z.B. Biomarker) einfach, schnell und kostengünstig charakterisiert werden.

Die Vorrichtung erlaubt die Bestimmung des Analyten in der flüssigen Probe mit einer hohen Detektionssensitivität, da der Analyt katalytisch über ein Enzym bzw. über Säure zu mindestens einem Gas umgesetzt wird und die Produktion bereits eines Mols Gas bei Normaldruck (1013 hPa) und Raumtemperatur (25°C) ein Volumen von 24,465 $\cdot$ $10^{-3}$ $m^3$ (d.h. ca. 24,465 Liter) einnimmt. Anders ausgedrückt wird bereits bei einer Umsetzung von einem Nanomol Analyt zu einem Nanomol Gas ein Gasvolumen von 24,465 Nanolitern freigesetzt, was über gängige optische Messverfahren ohne Weiteres messbar und quantifizierbar ist. Folglich verursacht die Gasproduktion eine Signalamplifikation, die eine hohe Detektionssensitivität bewirkt.

Ferner kann durch die erfindungsgemäße Vorrichtung eine hohe Detektionsspezifität bereitgestellt werden, da die Spezifität der enzymkatalysierten Umsetzungsreaktion von der Spezifität des Enzyms zu seinem Substrat abhängt und/oder da in einer bestimmten zu analysierenden Probe zwangsweise nur ein Analyt vorhanden sein kann, der sich durch eine Reaktion mit einer Säure zu einem Gas umwandelt (z.B. bei Blut als Probe: Umwandlung von $HCO_3^-$ zu $CO_2$ + $H_2O$). Im Falle einer Analyt-Bindung und Analyt-Umsetzung durch ein Enzym ist die Spezifität sogar höher als die Spezifität einer Vielzahl von Analyt-Antikörper-Bindungen, da anders als bei Analyt-Antikörper-Bindungen unspezifische Analyt-Enzym-Bindungen praktisch nicht bzw. kaum vorkommen. Selbst wenn der Analyt unspezifisch an das Enzym binden sollte, d.h. das Enzym nicht am aktiven Zentrum bindet, so kommt es zu keiner Umsetzung des Analyten und es wird kein Signal (in Form einer Produktion von Gas) generiert.

Darüberhinaus ist es durch die erfindungsgemäße Vorrichtung möglich, eine niedrige Detektionsvarianz bzw. eine hohe Präzision in der Detektion (d.h. eine hohe Nähe am wahren Wert), bereitzustellen, da die mindestens eine Fluidleitung der erfindungsgemäßen Vorrichtung dazu geeignet ist, die flüssige Probe über Kapillarkräfte und/oder über eine Mikropumpe zum Reaktionsbereich zu transportieren. Dies bewirkt, dass der für die erzeugte Signalhöhe verantwortliche kritische Schritt der Kontaktierung des Analyten mit dem Enzym und/oder der Säure "automatisiert" ist und in vorgegebenen Volumina erfolgt. Anders ausgedrückt entfallen hier Fehler, die durch manuelles Mischen bestimmter Volumina durch einen Benutzer bedingt werden (z.B. "Pipettierfehler"). Anders ausgedrückt ist die Mischgenauigkeit bestimmter Volumina bei der erfindungsgemäßen Vorrichtung von Versuch zu Versuch deutlich konstanter und die Varianz von Versuch zu Versuch deutlich kleiner. Ferner ist durch die Zugabe der flüssigen Probe auf den Aufnahmebereich der Vorrichtung der Start der Signalbildung exakt definiert ("automatisierter" Start) und somit Ungenauigkeiten bezüglich eines durch einen Benutzer manuell bewirkten Starts der Reaktion nicht gegeben. Folglich verkleinert sich die Varianz von Messung zu Messung und die Präzision der Detektion ist erhöht.

Die erfindungsgemäße Vorrichtung kann dadurch gekennzeichnet sein, dass sie mehrere Fluidleitungen enthält, bevorzugt mehrere Fluidleitungen, die dazu geeignet sind, über Kapillarkräfte und/oder mindestens eine Mikropumpe in der Fluidleitung, die flüssige Probe vom Aufnahmebereich über mindestens einen Enzymbereich und/oder mindestens einen Ansäuerungsbereich zum Reaktionsbereich zu transportieren. Hierbei können die Fluidleitungen jeweils mit dem mindestens einen Enzymbereich, Ansäuerungsbereich und/oder Reaktionsbereich der erfindungsgemäßen Vorrichtung verbunden sein oder alternativ jeweils jede Fluidleitung mit einem eigenen Enzymbereich, Ansäuerungsbereich und/oder Reaktionsbereich verbunden sein. In letzterem Fall ist mit der erfindungsgemäßen Vorrichtung die gleichzeitige Erfassung mehrerer, verschiedener Analyten möglich. Hierbei können die Fluidleitungen jeweils alle mit dem mindestens einen Aufnahmebereich der erfindungsgemäßen Vorrichtung verbunden sein oder alternativ jeweils mit einem eigenen Aufnahmebereich verbunden sein.

Die Vorrichtung kann mehrere Reaktionsbereiche enthalten, bevorzugt mehrere Reaktionsbereiche zur Ausbildung von Gasbläschen, wobei die Reaktionsbereiche jeweils eine Kammer enthalten oder daraus bestehen, die jeweils mit mindestens einer Fluidleitung fluidisch verbunden sind und jeweils flüssigkeitsdichte Wandungen aufweist.

Die Kammer kann mindestens eine Wandung, bevorzugt mindestens zwei gegenüberliegende Wandungen, enthalten, die eine Transparenz für Licht einer Wellenlänge in einem Bereich ausgewählt aus der Gruppe bestehend aus IR-Bereich, sichtbarer Bereich, UV-Bereich und Kombinationen hiervon aufweist, bevorzugt eine Transparenz für Licht einer Wellenlänge im sichtbaren Bereich.

Die Kammer kann ferner eine Wandung, bevorzugt mindestens zwei gegenüberliegende Wandungen, enthalten, die eine Dichtigkeit für Flüssigkeiten, Gase und Kombinationen hiervon, aufweist, bevorzugt eine Dichtigkeit für Flüssigkeiten.

Die Vorrichtung kann mehrere Kammern enthalten. In diesem Fall können die hier genannten Merkmale der Kammer für alle Kammern der Vorrichtung gelten.

Die Vorrichtung kann dadurch gekennzeichnet sein, dass der mindestens eine Aufnahmebereich der mindestens einen Fluidleitung zur Aufnahme einer flüssigen Probe ausgewählt aus der Gruppe bestehend aus wässrige Lösungen enthaltend oder bestehend aus Blut, Urin, Sputum, Nahrungsmittel, Flusswasser, Seewasser, Meerwasser, Grundwasser, Trinkwasser, Abwasser und Mischungen hiervon, geeignet ist.

Der mindestens eine Enzymbereich kann mindestens ein Enzym ausgewählt aus der Gruppe bestehend aus Urease, Laktatoxidase, Laktatdehydrogenase, Katalase, Pyruvatdecarboxylase, Thyreoperoxidase und Kombinationen hiervon enthalten.

Der mindestens eine Enzymbereich kann mindestens ein weiteres Enzym enthalten, wobei das mindestens eine weitere Enzym bevorzugt ausgewählt ist aus der Gruppe bestehend aus Catalase, Pyruvatdecarboxylase und Kombinationen hiervon.

Der mindestens eine Enzymbereich kann mindestens einen Cofaktor eines Enzyms enthalten, bevorzugt $NAD^+$.

Der mindestens eine Enzymbereich kann zwischen dem Aufnahmebereich zur Aufnahme einer flüssigen Probe und dem Reaktionsbereich zur Ausbildung von Gasbläschen angeordnet sein oder innerhalb des Reaktionsbereichs zur Ausbildung von Gasbläschen angeordnet sein. Falls die Vorrichtung mehrere Enzymbereiche aufweist kann eine solche Anordnung für alle Enzymbereiche der Vorrichtung gelten.

Der mindestens eine Enzymbereich kann das mindestens eine Enzym und/oder den mindestens einen Cofaktor des mindestens einen Enzyms in trockener Form, bevorzugt in lyophilisierter Form enthalten, oder in wässriger Form, bevorzugt als wässrige Lösung, wässrige Suspension oder wässriges Gel, enthalten.

Der mindestens eine Enzymbereich enthält biologische Zellen und/oder Zelllysat, wobei die biologischen Zellen und/oder das Zelllysat das mindestens eine Enzym enthalten, das dazu geeignet ist, die Umsetzung des zu bestimmenden Analyten zu mindestens einem Gas zu katalysieren. Der Vorteil an dieser Ausgestaltungsform ist, dass eine Isolation (Reinigung) des Enzyms nicht nötig ist und dass das Enzym in einer Umgebung ist, in der es eine höhere Langzeitstabilität als in gereinigter Form aufweisen kann. Folglich kann die Vorrichtung eine höhere Langzeitstabilität aufweisen und kann kostengünstiger bereitgestellt werden.

Der mindestens eine Enzymbereich kann mindestens Urease enthalten. Der mindestens eine Enzymbereich kann Laktatoxidase und Catalase enthalten. Der mindestens eine Enzymbereich kann Pyruvatdecarboxylase enthalten. Der mindestens eine Enzymbereich kann Laktatdehydrogenase und Pyruvatdecarboxylase enthalten.

Die mindestens eine Fluidleitung kann eine Membran enthalten, die bevorzugt einen Porendurchmesser von $\leq 20$ μm, bevorzugt $\leq 6$ μm, besonders bevorzugt $\leq 2$ μm, insbesondere $\leq 100$ nm, optional $\leq 1$ nm, aufweist. Die Membran kann zur Abtrennung biologischer Zellen geeignet sein, bevorzugt zur Abtrennung von Blutzellen. Die Membran kann zwischen dem Aufnahmebereich zur Aufnahme einer flüssigen Probe und dem Reaktionsbereich zur Ausbildung von Gasbläschen angeordnet sein, bevorzugt zwischen dem Aufnahmebereich zur Aufnahme einer flüssigen Probe und dem Enzymbereich und/oder dem Ansäuerungsbereich. Falls die Vorrichtung mehrere Membranen aufweist kann eine solche Anordnung für alle Membranen der Vorrichtung gelten.

Der mindestens eine Ansäuerungsbereich enthält eine Säure, die ausgewählt ist aus der Gruppe bestehend aus bei Raumtemperatur und Normaldruck feste Säuren, besonders bevorzugt Citronensäure, Ascorbinsäure, Apfelsäure, Stearinsäure, Palmitinsäure, Myristinsäure, Laurinsäure und Mischungen hiervon

Der mindestens eine Ansäuerungsbereich ist bevorzugt zwischen dem Aufnahmebereich zur Aufnahme einer flüssigen Probe und dem Reaktionsbereich zur Ausbildung von Gasbläschen angeordnet, besonders bevorzugt innerhalb des Reaktionsbereichs zur Ausbildung von Gasbläschen. Falls die Vorrichtung mehrere Ansäuerungsbereiche aufweist kann eine solche Anordnung für alle Ansäuerungsbereiche der Vorrichtung gelten.

Die Vorrichtung, bevorzugt die mindestens eine Fluidleitung, kann mindestens einen Oxidationsbereich enthalten, wobei der mindestens eine Oxidationsbereich mindestens ein Oxidationsmittel enthält, bevorzugt mindestens ein Oxidationsmittel und mindestens eine Säure.

Der mindestens eine Oxidationsbereich enthält bevorzugt ein Oxidationsmittel, das ausgewählt ist aus der Gruppe bestehend aus Kaliumpermanganat, Mangandioxid, $NAD^+$ und Mischungen hiervon.

Der mindestens eine Oxidationsbereich enthält bevorzugt eine Säure, die ausgewählt ist aus der Gruppe bestehend aus bei Raumtemperatur und Normaldruck feste Säuren, besonders bevorzugt Citronensäure, Ascorbinsäure, Apfelsäure, Stearinsäure, Palmitinsäure, Myristinsäure, Laurinsäure und Mischungen hiervon.

Der mindestens eine Oxidationsbereich ist bevorzugt zwischen dem Aufnahmebereich zur Aufnahme einer flüssigen Probe und dem Reaktionsbereich zur Ausbildung von Gasbläschen angeordnet, besonders bevorzugt zwischen dem Enzymbereich und dem Reaktionsbereich zur Ausbildung von Gasbläschen. Insbesondere überlappt der mindestens eine Oxidationsbereich mit dem Ansäuerungbereich zumindest bereichsweise oder ist mit diesem identisch.

Die mindestens eine Fluidleitung kann eine Länge von 0,1 bis 20 cm, bevorzugt 0,5 bis 10 cm, besonders bevorzugt 1 bis 5 cm, aufweisen. Die mindestens eine Fluidleitung kann eine Breite von 0,05 bis 20 mm, besonders bevorzugt 0,1 bis 10 mm, besonders bevorzugt 1 bis 5 mm, insbesondere 2 bis 4 mm, aufweisen. Die mindestens eine Fluidleitung kann eine Höhe von 0,05 bis 2 mm, besonders bevorzugt 0,1 bis 1 mm, besonders bevorzugt 0,2 bis 0,8 mm, insbesondere 0,4 bis 0,6 mm, aufweisen. Die mindestens eine Fluidleitung kann einen maximalen Durchmesser im Bereich von 0,05 bis 20 mm, bevorzugt 0,1 bis 10 mm, besonders bevorzugt 1 bis 5 mm, insbesondere 2 bis 4 mm, aufweisen.

Der bestimmte Analyt kann ausgewählt sein aus der Gruppe bestehend aus kleines organisches Molekül mit einer Masse von < 500 Da, Peptid, Protein und Mischungen hiervon, bevorzugt ausgewählt ist aus der Gruppe bestehend aus Hormon, Stoffwechselprodukt mit einer Masse von < 500 Da, Kohlenhydrat, Sterol, Triglycerid Carbonsäure, Amid-Derivat von einer Carbonsäure und Mischungen hiervon, besonders bevorzugt ausgewählt aus der Gruppe bestehend aus T3-Hormon, T4-Hormon, Glukose, Cholesterin, Triglyceride aus dem Blut Milchsäure, Harnstoff und Mischungen hiervon.

Der bestimmte Analyt kann ein Analyt sein, der ein Marker für einen Zustand ist, der ausgewählt ist aus der Gruppe bestehend aus Erkrankung, Wasserverschmutzung, Lebensmittelverunreinigung, und Kombinationen hiervon.

Die Vorrichtung kann in oder an einem optischen Detektionsgerät angeordnet sein, bevorzugt in oder an einem optisches Mikroskop, wobei das optische Detektionsgerät (z.B. das optische Mikroskop) besonders bevorzugt dazu konfiguriert ist, den Reaktionsbereich der Vorrichtung optisch zu detektieren und eine qualitative und/oder quantitative Bestimmung der Konzentration des Analyts in der Probe durchzuführen.

Das optische Detektionsgerät (z.B. das optische Mikroskop) kann dazu konfiguriert sein, qualitativ ein Vorhandensein des Analyten in der Probe zu bestimmen, wenn es im Reaktionsbereich zur Ausbildung von Gasbläschen kommt.

Das optische Detektionsgerät (z.B. das optische Mikroskop) kann dazu konfiguriert sein, quantitativ die Konzentration des Analyten in der Probe über eine Anzahl und ein Volumens der Gasbläschen pro Zeiteinheit zu bestimmen, insbesondere über den Zusammenhang, dass das Produkt aus Anzahl und Volumen der Gasbläschen pro Zeiteinheit direkt proportional ist zu der Konzentration des Analyts in der Probe.

Das optische Detektionsgerät kann eine Energiequelle enthalten, die dazu konfiguriert ist, die erfindungsgemäße Vorrichtung (z.B. mindestens eine Mikropumpe hiervon) mit Energie zu versorgen. Diese Energiequelle ist bevorzugt ausgewählt aus Energie aus einem Stromnetz, Batterie, Akkumulator, photovoltaisches Element und Kombinationen hiervon.

Ferner wird erfindungsgemäß ein Verfahren zum Nachweis eines bestimmten Analyten in einer flüssigen Probe über enzymkatalysierte und/oder säurevermittelte Umsetzung des Analyten zu mindestens einem Gas bereitgestellt, umfassend die Schritte

a) Aufbringen einer flüssigen Probe, die möglicherweise den zu bestimmenden Analyten enthält, auf einen Aufnahmebereich der erfindungsgemäßen Vorrichtung;
b) Optische Detektion des Reaktionsbereichs der erfindungsgemäßen Vorrichtung zu einem Zeitpunkt, an dem die Enzym-haltige und/oder Säure-haltige flüssige Probe von der Fluidleitung zu dem Reaktionsbereich transportiert wurde; und
c) Bestimmung, dass der bestimmte Analyt in der Probe vorhanden ist, wenn es im Reaktionsbereich zur Ausbildung von Gasbläschen kommt.

Das Verfahren kann dadurch gekennzeichnet sein, dass die optische Detektion über ein optisches Detektionsgerät ausgewählt aus der Gruppe bestehend aus Kamera, Mikroskop, Photometer, Refraktometer und Kombinationen hiervon erfolgt, bevorzugt über ein Mikroskop.

Das Verfahren kann eine quantitative Bestimmung der Konzentration des Analyts in der Probe umfassen, bevorzugt über eine Bestimmung der Anzahl und des Volumens der Gasbläschen pro Zeiteinheit, besonders bevorzugt über den Zusammenhang, dass das Produkt aus Anzahl und Volumen der Gasbläschen pro Zeiteinheit direkt proportional ist zu der Konzentration des Analyts in der Probe.

Das kann die folgenden Schritte umfassen:

a) Aufbringen mindestens einer weiteren flüssigen Probe enthaltend eine bekannte Konzentration des zu bestimmenden Analyten auf einen Aufnahmebereich einer Vorrichtung gemäß einem der vorhergehenden Ansprüche;

b) Optische Detektion des Reaktionsbereichs der Vorrichtung gemäß einem der vorhergehenden Ansprüche zu einem Zeitpunkt, an dem an dem die Enzym-haltige und/oder Säure-haltige flüssige Probe von der Fluidleitung zu dem Reaktionsbereich transportiert wurde; und

c) Quantitative Bestimmung der Konzentration des Analyts in der Probe, bevorzugt über eine Bestimmung der Anzahl und des Volumens der Gasbläschen pro Zeiteinheit, besonders bevorzugt über den Zusammenhang, dass das Produkt aus Anzahl und Volumen der Gasbläschen pro Zeiteinheit direkt proportional ist zu der Konzentration des Analyts in der Probe.

Darüberhinaus wird die Verwendung der erfindungsgemäßen Vorrichtung zur in-vitro-Diagnose einer Krankheit vorgeschlagen, wobei die flüssige Probe bevorzugt ausgewählt ist aus der Gruppe bestehend aus Blut, Urin, Sputum und Mischungen hiervon. Ferner wird die Verwendung der erfindungsgemäßen Vorrichtung zur Qualitätskontrolle von Lebensmitteln, bevorzugt zur Qualitätskontrolle einer Lebensmittelflüssigkeit, besonders bevorzugt zur Qualitätskontrolle von Wein, Fruchtsäften und Kombinationen hiervon, vorgeschlagen. Zudem wird die Verwendung der erfindungsgemäßen Vorrichtung zur Untersuchung einer Qualität von Wasser, bevorzugt einer Qualität von Flusswasser, Seewasser, Meerwasser, Trinkwasser und Kombinationen hiervon, vorgeschlagen.

Anhand der nachfolgenden Figuren und Beispiele soll der erfindungsgemäße Gegenstand näher erläutert werden.

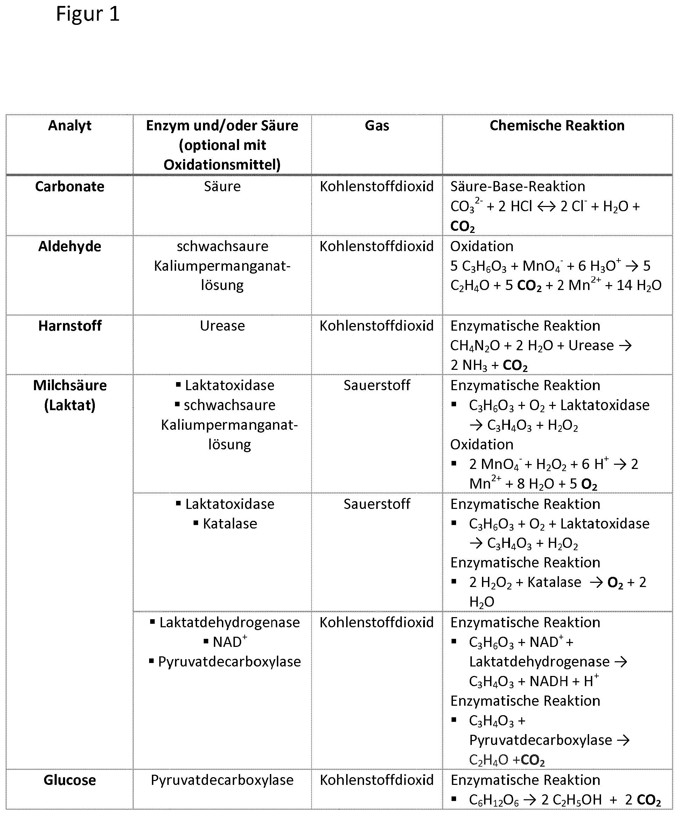

Figur 1 zeigt beispielhaft mögliche Enzyme und/oder Säuren (optional mit Oxidationsmittel) zur Umsetzung von bestimmten Analyten (Biomarkern), welche von der Vorrichtung enthalten sein können und in dem Verfahren verwendet werden können. Insbesondere stellt die Figur auch die jeweiligen chemischen Reaktionen dar, welche der Gasentwicklung zu Grunde liegen.

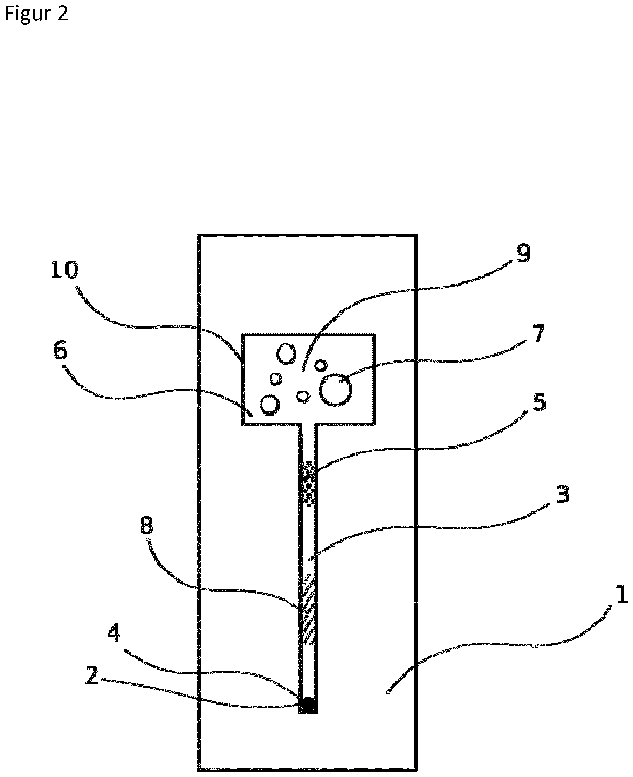

Figur 2 zeigt beispielhaft eine mögliche Vorrichtung 1 zum Nachweis eines bestimmten Analyten in einer flüssigen Probe 2 über enzymkatalysierte Umsetzung des Analyten zu mindestens einem Gas 7. Die Vorrichtung 1 enthält mindestens eine Fluidleitung 3, die fluidisch mit mindestens einem Aufnahmebereich 4 zur Aufnahme einer flüssigen Probe 2 (z.B. Blut) und fluidisch mit mindestens einem Enzymbereich 5 verbunden ist, wobei der Enzymbereich 5 mindestens ein bestimmtes Enzym enthält, das die Umsetzung des zu bestimmenden Analyten zu mindestens einem Gas 7 katalysiert. Ferner enthält die Vorrichtung 1 mindestens einen Reaktionsbereich 6 zur Ausbildung von Gasbläschen 7, wobei der Reaktionsbereich 6 eine fluidische Verbindung zur Fluidleitung 3 aufweist und ansonsten von einer gasdichten Wand 10 begrenzt ist. Die Vorrichtung 1 ist dadurch gekennzeichnet, dass die mindestens eine Fluidleitung 3 dazu geeignet ist, die flüssige Probe 2 vom Aufnahmebereich 4 über Kapillarkräfte zum Reaktionsbereich 6 zu transportieren, wobei hierbei das mindestens eine bestimmte Enzym vom Enzymbereich 5 zumindest teilweise mit in den Reaktionsbereich 6 transportiert wird. Bei dieser Ausgestaltungsform enthält die Vorrichtung 1 ferner zwischen dem Aufnahmebereich 4 und dem Enzymbereich 5 eine Membran 8, die zur Abtrennung biologischer Zellen geeignet ist (z. B. eine Membran zur Separation von Blutzellen von Blutplasma). Diese Membran 8 stellt sicher, dass keine biologischen Zellen über Kapillarkräfte von der Fluidleitung 3 bis zum Enzymbereich 5 gelangen. Zudem enthält diese Vorrichtung 1 noch einen Ansäuerungsbereich 9, der mindestens eine Säure (z.B. HCl) enthält und in diesem Fall mit dem Reaktionsbereich 6 zusammenfällt. Der Ansäuerungsbereich 9 stellt sicher, dass die flüssige Probe, die im Reaktionsbereich 6 Enzym enthält, angesäuert wird. Im Fall von Gasen, die sich unter Säurebildung in der flüssigen Probe lösen (z.B. Bildung von $H_2CO_3$ bei dem Gas $CO_2$) wird durch die Ansäuerung das Gleichgewicht in Richtung Gasbildung verschoben und damit erfolgt eine stärkere (quantitative) Ausgasung von $CO_2$. Kurz gesagt kann das Vorhandensein des Ansäuerungsbereichs 9 bei dieser Art von Gasen die Detektionssensitivität deutlich erhöhen.

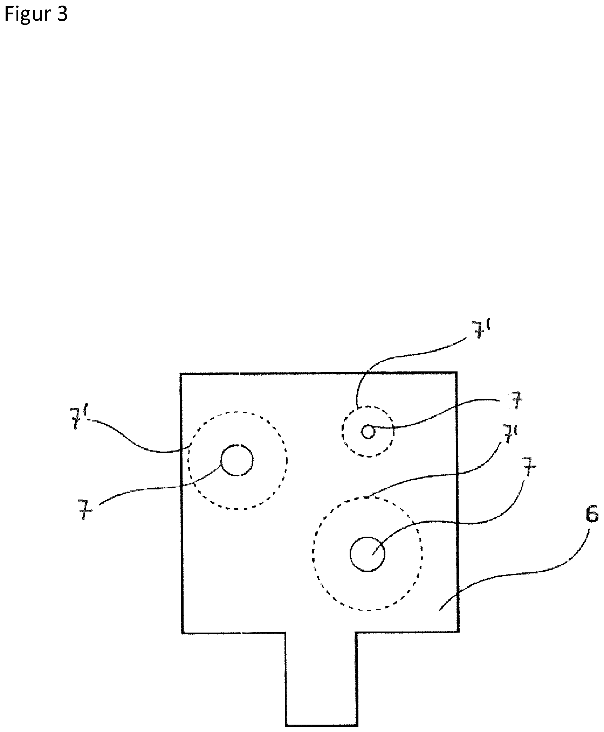

Figur 3 zeigt eine Draufsicht auf eine Reaktionskammer 6 einer Vorrichtung. Es ist ein zeitlicher Verlauf während der Reaktion dargestellt, wobei die Gasbläschen 7 im lauf der Zeit zu größeren Gasbläschen 7' anwachsen. Neben der endgültigen Volumenausdehnung der Gasbläschen 7 zu den größeren Gasbläschen 7' kann auch die zeitliche Volumenzunahme vom Zustand der ursprünglichen Gasbläschen 7 bis zum Zustand der größeren Gasbläschen 7' ausgenutzt werden.

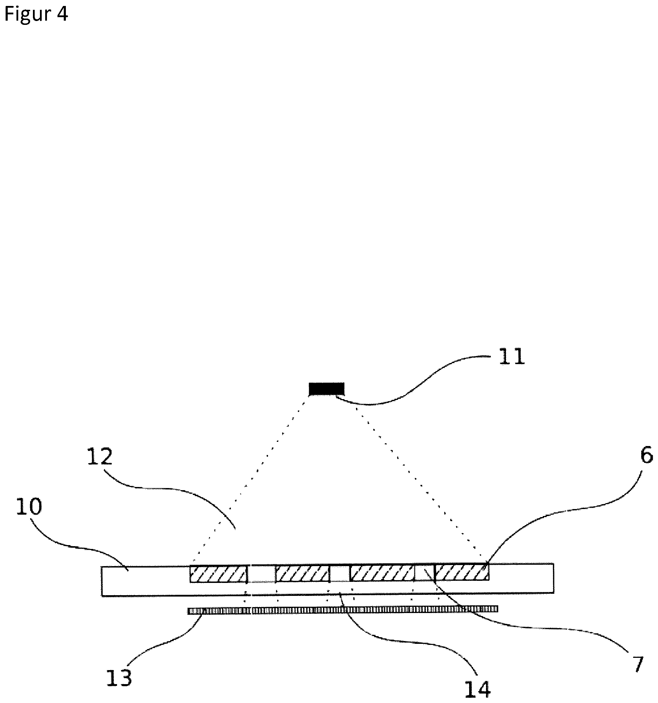

Figur 4 zeigt einen Querschnitt eines Detektionsgeräts, welches dazu geeignet ist, eine optische Aufnahme des Reaktionsbereichs der erfindungsgemäßen Vorrichtung vorzunehmen und eine quantitative Bestimmung der Konzentration des Analyts in der Probe durchzuführen und auszugeben. Die Gasbläschen 7, welche sich im Reaktionsbereich 6 der erfindungsgemäßen Vorrichtung ausbilden, werden durch elektromagnetische Strahlung 12 einer im Wesentlichen kohärenten oder nicht-kohärenten Lichtquelle 11 (z.B. eine LED, eine OLED, ein Laser und/oder eine Gasdrucklampe) vollständig beleuchtet. Ein Bildsensor 13 (z.B. in Form eines Diodenarrays), welcher sich unter dem Reaktionsbereich 6 befindet, nimmt das durch die Gasbläschen 7 erzeugte optische Bild 14 (d.h. deren charakteristisches Interferenzmuster) auf. Über ein Datenverarbeitungsprogramm kann aus diesem optischen Bild 14 das Produkt aus Anzahl und Volumen der Gasbläschen pro Zeiteinheit errechnet werden, welches direkt proportional ist zur Umsatzgeschwindigkeit und zur Konzentration des Analyten in der Probe. Zwischen dem Bildsensor und dem Reaktionsbereich kann sich ebenfalls noch eine optische Vorrichtung befinden (hier nicht dargestellt).

**Beispiel 1 - Grundlagen zum Verfahren zum Nachweis eines bestimmten Analyten in einer flüssigen Probe**

Die maximale Umsatzgeschwindigkeit $v_{max}$ einer enzymatischen Reaktion hängt ab von der Temperatur und dem pH-Wert der Lösung, in welcher die Reaktion durchgeführt wird. Für einen bestimmten, konstanten pH-Wert ist $v_{max}$ abhängig von der Umgebungstemperatur der erfindungsgemäßen Vorrichtung. Ist auch die Umgebungstemperatur konstant (z.B. konstante 25 °C Raumtemperatur) so nimmt $v_{max}$ einen ganz bestimmten Wert ein. In diesem Fall gilt, dass die gemessene Umsatzgeschwindigkeit v von Substrat (Analyt) abhängig ist von der Konzentration von Substrat (Analyt) in der Lösung (Michaelis-Menten-Therorie). Es gilt hierbei die Gleichung

$$v = (v_{max} \cdot [Analyt]) / (k_m + [Analyt])$$

wobei [Analyt] die Analytkonzentration darstellt und $k_m$ die Michaelis-Menten-Konstante des Enzyms repräsentiert.

Bei bekanntem $v_{max}$ unter bestimmten Bedingungen und bekannter Michaelis-Menten-Konstante $k_m$ kann somit über die Messung der Umsatzgeschwindigkeit des Substrats (Analyten) auf die Konzentration an Substrat (Analyt) in der flüssigen Probe rückgeschlossen werden.

Die Umsatzgeschwindigkeit ist direkt proportional zum produzierten Gasvolumen pro Zeiteinheit, also proportional zum Produkt aus Anzahl und Volumen detektierter Gasbläschen. Es gilt also der Zusammenhang

$$v \sim [Anzahl(Gasbläschen) \cdot Volumen(Gasbläschen)]$$

Viele der entstehenden Gasbläschen lösen sich schlecht in der flüssigen Probe, sodass der oben genannte Zusammenhang uneingeschränkt gilt.

Da Kohlenstoffdioxid in wässriger Lösung sehr gut löslich ist und zum Teil zu Kohlensäure dissoziiert, nutzt man die Änderung des pH-Wertes zum Umwandeln in den gasförmigen Aggregatzustand. Da die Probenkammer in alle drei Dimensionen von einer Gas undurchlässigen Schicht begrenzt wird, bilden sich Gasblasen, welche nicht aus der Probenkammer entweichen können. Die Menge und Größe der Gasblasen wird mithilfe einer optischen Methode untersucht. Die Menge und Größe der Gasblasen korreliert mit Quantität des Analyts in der Probe.

**Beispiel 2 - Verfahren zum Nachweis von Harnstoff in einer flüssigen Probe**

Zum Nachweis des Analyten Harnstoff enthält die Vorrichtung das Enzym Urease in dem Enzymbereich. Wie in Figur 1 ersichtlich wird durch das Enzym Urease die Umsetzung von Harnstoff zu den beiden Gasen Ammoniak und Kohlenstoffdioxid katalysiert.

Durch einen Ansäuerungsbereich auf der Vorrichtung wird sichergestellt, dass einerseits das entstehende Kohlenstoffdioxid nicht als $H_3O^+$ und $HCO_3^-$ in Lösung geht, sondern quantitativ ausgast. Dadurch wird die Sensitivität der Detektion der Vorrichtung für Harnstoff erhöht.

Die Ansäuerung bewirkt zudem, dass der entstehende Ammoniak bevorzugt als $NH_4^+$ und $OH^-$ in Lösung geht. Da Ammoniak aber ohnehin auch bei neutralem pH eine hohe Tendenz zur Lösung in wässrigen Medien aufweist wird durch die Ansäuerung das Gleichgewicht kaum in Richtung gelöstem Ammoniak verschoben. Anders ausgedrückt verringert die Ansäuerung die Produktion von Ammoniakgas kaum, sodass bezüglich der Herstellung von Ammoniakgas die Ansäuerung kaum einen negativen Einfluss auf die Detektionssensitivität bewirkt.

Das Produkt aus Anzahl und Volumen (Menge) der Kohlendioxid-Gasblasen ist somit anhängig von der Harnstoffkonzentration in der eingesetzten flüssigen Probe. Die Menge an produzierten Gasblasen nimmt mit sinkender Harnstoffkonzentration ab.

Die Menge der Gasblasen kann über ein softwaregesteuertes Mikroskop aufgezeichnet werden und ausgewertet werden (z.B. bzgl. Ihrer Anzahl und ihrer geometrischen Eigenschaften).

Beispielhaft sei die Anwendung der Vorrichtung in der sog. "point of care"-

Diagnostik von Harnstoff beschrieben:

Ein Tropfen Blut wird auf den mindestens einen Aufnahmebereich der Vorrichtung aufgebracht. Der Tropfen kann beispielsweise direkt von einem (frisch) punktierten Finger einer Person aufgenommen werden. Die Vorrichtung enthält in diesem Fall vorteilhafterweise eine plasmaseparierende Membran, sodass die Blutzellen zurückgehalten werden und nur Blutplasma bis zum Enzymbereich vordringen kann. Der Enzymbereich enthält vorteilhafterweise das Enzym Urease in lyophilisierter Form, da in dieser Form die Langzeitstabilität des Enzyms sehr hoch ist und dadurch auch die Vorrichtung eine lange Verwendbarkeit sicherstellt.

Das Blutplasma wird vom Aufnahmebereich über Kapillarkräfte entlang der Fluidleitung zum Enzymbereich gezogen, trifft dort auf die lyophilisierte Urease, die im Blutplasma in Lösung geht. Die Mischung aus Blutplasma und Urease wird durch Kapillarkraft prompt über den Ansäuerungsbereich zu dem Reaktionsbereich transportiert, wo Kohlendioxid durch die Zersetzung von Harnstoff freigesetzt wird. Die Menge der gebildeten Gasbläschen wird mithilfe einer optischen Methode (z.B. ein softwaregesteuertes Mikroskop) aufgezeichnet, was Rückschlüsse über die Konzentration von Harnstoff in dem eingesetzten Blutstropfen erlaubt.

**Beispiel 3 -Verfahren zum Nachweis von Laktat in einer flüssigen Probe**

Das Enzym Laktatoxidase wandelt Laktat selektiv in Pyruvat und Wasserstoffperoxid um (siehe Figur 1). In einem zweiten Schritt reagiert das Wasserstoffperoxid durch seine stark reduzierende Wirkung mit einer leicht schwefelsaurem Kaliumpermanganatlösung. Die Redoxreaktion führt zum einem zur Entfärbung der stark violett gefärbten Kaliumpermangantlösung und zum anderen zur Bildung von Sauerstoff (siehe Figur 1).

Für Reaktion des Wasserstoffperoxids mit der schwefelsäuren Kaliumpermanganatlösung benötigt die Vorrichtung somit ferner einen Oxidationsbereich, der mindestens ein Oxidationsmittel (z.B. $MnO_4^-$ durch gelöstes $KMnO_4$) und mindestens eine Säure ($H_3O^+$ z.B. durch gelöstes $H_2SO_4$) enthält.

Das Produkt aus Anzahl und Volumen (Menge) der Sauerstoff-Gasblasen ist somit abhängig von der Laktatkonzentration in der eingesetzten flüssigen Probe. Die Menge an produzierten Gasblasen nimmt mit sinkender Laktatkonzentration ab.

Die Menge der Gasblasen kann über ein softwaregesteuertes Mikroskop aufgezeichnet werden und ausgewertet werden (z.B. bzgl. Ihrer Anzahl und ihrer geometrischen Eigenschaften).

**Patentansprüche**

**1.** Vorrichtung zum Nachweis eines bestimmten Analyten in einer flüssigen Probe über enzymkatalysierte und/oder säurevermittelte Umsetzung des Analyten zu mindestens einem Gas, enthaltend

a) mindestens eine Fluidleitung;

b) mindestens einen Aufnahmebereich zur Aufnahme einer flüssigen Probe;

c) i) mindestens einen Enzymbereich, der mindestens ein bestimmtes Enzym enthält, das dazu geeignet ist, die Umsetzung des zu bestimmenden Analyten zu mindestens einem Gas zu katalysieren, wobei der mindestens eine Enzymbereich biologische Zellen und/oder Zelllysat enthält, wobei die biologischen Zellen und/oder das Zelllysat das mindestens eine Enzym enthalten, das dazu geeignet ist, die Umsetzung des zu bestimmenden Analyten zu mindestens einem Gas zu katalysieren; und/oder ii) mindestens einen Ansäuerungsbereich, der mindestens eine Säure enthält, die ausgewählt ist aus der Gruppe bestehend aus bei Raumtemperatur und Normaldruck festen Säuren; und

d) mindestens einen Reaktionsbereich zur Ausbildung von Gasbläschen, wobei der Reaktionsbereich eine Kammer enthält oder daraus besteht, die mit der mindestens einen Fluidleitung fluidisch verbunden ist und flüssigkeitsdichte Wandungen aufweist; **dadurch gekennzeichnet, dass** die mindestens eine Fluidleitung dazu geeignet ist, über Kapillarkräfte und/oder mindestens eine Mikropumpe in der Fluidleitung, die flüssige Probe vom Aufnahmebereich über den mindestens einen Enzymbereich und/oder den mindestens einen Ansäuerungsbereich zum Reaktionsbereich zu transportieren.

**2.** Vorrichtung gemäß Anspruch 1, **dadurch gekennzeichnet, dass** die Vorrichtung

i) mehrere Fluidleitungen enthält, bevorzugt mehrere Fluidleitungen, die dazu geeignet sind, über Kapillarkräfte und/oder mindestens eine Mikropumpe in der Fluidleitung, die flüssige Probe vom Aufnahmebereich über mindestens einen Enzymbereich und/oder mindestens einen Ansäuerungsbereich zum Reaktionsbereich zu transportieren; und/oder

ii) mehrere Reaktionsbereiche enthält, bevorzugt mehrere Reaktionsbereiche zur Ausbildung von Gasbläschen, wobei die Reaktionsbereiche jeweils eine Kammer enthalten oder daraus bestehen, die jeweils mit mindestens einer Fluidleitung fluidisch verbunden sind und jeweils flüssigkeitsdichte Wandungen aufweist.

3. Vorrichtung gemäß einem der vorhergehenden Ansprüche, **dadurch gekennzeichnet, dass** die Kammer mindestens eine Wandung, bevorzugt mindestens zwei gegenüberliegende Wandungen, enthält, die

i) eine Transparenz für Licht einer Wellenlänge in einem Bereich ausgewählt aus der Gruppe bestehend aus IR-Bereich, sichtbarer Bereich, UV-Bereich und Kombinationen hiervon aufweist, bevorzugt eine Transparenz für Licht einer Wellenlänge im sichtbaren Bereich aufweist; und/oder
ii) eine Dichtigkeit für Flüssigkeiten, Gase und Kombinationen hiervon, aufweist, bevorzugt eine Dichtigkeit für Flüssigkeiten aufweist.

4. Vorrichtung gemäß einem der vorhergehenden Ansprüche, **dadurch gekennzeichnet, dass** der mindestens eine Aufnahmebereich zur Aufnahme einer flüssigen Probe ausgewählt aus der Gruppe bestehend aus wässrige Lösungen enthaltend oder bestehend aus Blut, Urin, Sputum, Nahrungsmittel, Flusswasser, Seewasser, Meerwasser, Grundwasser, Trinkwasser, Abwasser und Mischungen hiervon, geeignet ist.

5. Vorrichtung gemäß einem der vorhergehenden Ansprüche, **dadurch gekennzeichnet, dass** der mindestens eine Enzymbereich

i) mindestens ein Enzym ausgewählt aus der Gruppe bestehend aus Urease, Laktatoxidase, Laktatdehydrogenase, Katalase, Pyruvatdecarboxylase, Thyreoperoxidase und Kombinationen hiervon enthält; und/oder
ii) mindestens ein weiteres Enzym enthält, wobei das mindestens eine weitere Enzym bevorzugt ausgewählt ist aus der Gruppe bestehend aus Catalase, Pyruvatdecarboxylase und Kombinationen hiervon;
iii) mindestens einen Cofaktor eines Enzyms enthält, bevorzugt $NAD^+$;
und/oder
iv) zwischen dem Aufnahmebereich zur Aufnahme einer flüssigen Probe und dem Reaktionsbereich zur Ausbildung von Gasbläschen angeordnet ist oder innerhalb des Reaktionsbereichs zur Ausbildung von Gasbläschen angeordnet ist; und/oder
v) das mindestens eine Enzym und/oder mindestens einen Cofaktor des mindestens einen Enzyms in trockener Form, bevorzugt in lyophilisierter Form, enthält, oder in wässriger Form, bevorzugt als wässrige Lösung, wässrige Suspension oder wässriges Gel, enthält.

6. Vorrichtung gemäß einem der vorhergehenden Ansprüche, **dadurch gekennzeichnet, dass** die mindestens eine Fluidleitung eine Membran enthält, die bevorzugt

i) einen Porendurchmesser von $\leq 20\ \mu m$, bevorzugt $\leq 6\ \mu m$, besonders bevorzugt $\leq 2\ \mu m$, insbesondere $\leq 100$ nm, optional $\leq 1$ nm, aufweist; und/oder
ii) zur Abtrennung biologischer Zellen, bevorzugt zur Abtrennung von Blutzellen, geeignet ist; und/oder
iii) zwischen dem Aufnahmebereich zur Aufnahme einer flüssigen Probe und dem Reaktionsbereich zur Ausbildung von Gasbläschen angeordnet ist, bevorzugt zwischen dem Aufnahmebereich zur Aufnahme einer flüssigen Probe und dem Enzymbereich und/oder dem Ansäuerungsbereich.

7. Vorrichtung gemäß einem der vorhergehenden Ansprüche, **dadurch gekennzeichnet, dass** der Ansäuerungsbereich

i) eine Säure enthält, die ausgewählt ist aus der Gruppe bestehend aus Citronensäure, Ascorbinsäure, Apfelsäure, Stearinsäure, Palmitinsäure, Myristinsäure, Laurinsäure und Mischungen hiervon; und/oder der
ii) zwischen dem Aufnahmebereich zur Aufnahme einer flüssigen Probe und dem Reaktionsbereich zur Ausbildung von Gasbläschen angeordnet ist oder innerhalb des Reaktionsbereichs zur Ausbildung von Gasbläschen angeordnet ist.

8. Vorrichtung gemäß einem der vorhergehenden Ansprüche, **dadurch gekennzeichnet, dass** die Vorrichtung, bevorzugt die mindestens eine Fluidleitung, mindestens einen Oxidationsbereich enthält, der mindestens ein Oxidationsmittel enthält, bevorzugt mindestens ein Oxidationsmittel und mindestens eine Säure, wobei der mindestens eine Oxidationsbereich bevorzugt

i) ein Oxidationsmittel enthält, das ausgewählt ist aus der Gruppe bestehend aus Mangandioxid, Kaliumpermanganat, $NAD^+$ und Mischungen hiervon; und/oder der

ii) eine Säure enthält, die ausgewählt ist aus der Gruppe bestehend aus bei Raumtemperatur und Normaldruck feste Säuren, besonders bevorzugt Citronensäure, Ascorbinsäure, Apfelsäure, Stearinsäure, Palmitinsäure, Myristinsäure, Laurinsäure und Mischungen hiervon; und/oder

iii) zwischen dem Aufnahmebereich zur Aufnahme einer flüssigen Probe und dem Reaktionsbereich zur Ausbildung von Gasbläschen angeordnet ist, besonders bevorzugt zwischen dem Enzymbereich und dem Reaktionsbereich zur Ausbildung von Gasbläschen, wobei der mindestens eine Oxidationsbereich insbesondere mit dem Ansäuerungsbereich zumindest bereichsweise überlappt oder mit diesem identisch ist.

9. Vorrichtung gemäß einem der vorhergehenden Ansprüche, **dadurch gekennzeichnet, dass** der bestimmte Analyt

i) ausgewählt ist aus der Gruppe bestehend aus kleines organisches Molekül mit einer Masse von < 500 Da, Peptid, Protein und Mischungen hiervon, bevorzugt ausgewählt ist aus der Gruppe bestehend aus Hormon, Stoffwechselprodukt mit einer Masse von < 500 Da, Kohlenhydrat, Sterol, Triglycerid Carbonsäure, Amid-Derivat von einer Carbonsäure und Mischungen hiervon, besonders bevorzugt ausgewählt aus der Gruppe bestehend aus T3-Hormon, T4-Hormon, Glukose, Cholesterin, Triglyceride aus dem Blut Milchsäure, Harnstoff und Mischungen hiervon; und/oder

ii) ein Analyt ist, der ein Marker für einen Zustand ist, der ausgewählt ist aus der Gruppe bestehend aus Erkrankung, Wasserverschmutzung, Lebensmittelverunreinigung, und Kombinationen hiervon.

10. Vorrichtung gemäß einem der vorhergehenden Ansprüche, **dadurch gekennzeichnet, dass** die Vorrichtung in oder an einem optischen Detektionsgerät angeordnet ist, bevorzugt ein optisches Mikroskop, wobei das optische Detektionsgerät besonders bevorzugt dazu konfiguriert ist, den Reaktionsbereich der Vorrichtung optisch zu detektieren und eine qualitative und/oder quantitative Bestimmung der Konzentration des Analyts in der Probe durchzuführen, wobei das Detektionsgerät insbesondere dazu konfiguriert ist,

i) qualitativ ein Vorhandenseins des Analyten in der Probe zu bestimmen, wenn es im Reaktionsbereich zur Ausbildung von Gasbläschen kommt; und/oder

ii) quantitativ die Konzentration des Analyten in der Probe über eine Anzahl und ein Volumens der Gasbläschen pro Zeiteinheit zu bestimmen, insbesondere über den Zusammenhang, dass das Produkt aus Anzahl und Volumen der Gasbläschen pro Zeiteinheit direkt proportional ist zu der Konzentration des Analyts in der Probe.

11. Verfahren zum Nachweis eines bestimmten Analyten in einer flüssigen Probe über enzymkatalysierte und/oder säurevermittelte Umsetzung des Analyten zu mindestens einem Gas, umfassend die Schritte

a) Aufbringen einer flüssigen Probe, die möglicherweise den zu bestimmenden Analyten enthält, auf einen Aufnahmebereich einer Vorrichtung gemäß einem der vorhergehenden Ansprüche;

b) Optische Detektion des Reaktionsbereichs der Vorrichtung gemäß einem der vorhergehenden Ansprüche zu einem Zeitpunkt, an dem die Enzym-haltige und/oder Säure-haltige flüssige Probe von der Fluidleitung zu dem Reaktionsbereich transportiert wurde; und

c) Bestimmung, dass der bestimmte Analyt in der Probe vorhanden ist, wenn es im Reaktionsbereich zur Ausbildung von Gasbläschen kommt.

12. Verfahren gemäß Anspruch 11, **dadurch gekennzeichnet, dass** die optische Detektion über ein optisches Detektionsgerät ausgewählt aus der Gruppe bestehend aus Kamera, Mikroskop, Photometer, Refraktometer und Kombinationen hiervon erfolgt, bevorzugt über ein Mikroskop.

13. Verfahren gemäß einem der Ansprüche 11 oder 12, **dadurch gekennzeichnet, dass** das Verfahren eine quantitative Bestimmung der Konzentration des Analyts in der Probe umfasst, bevorzugt über eine Bestimmung der Anzahl und des Volumens der Gasbläschen pro Zeiteinheit, besonders bevorzugt über den Zusammenhang, dass das Produkt aus Anzahl und Volumen der Gasbläschen pro Zeiteinheit direkt proportional ist zu der Konzentration des Analyts in der Probe.

14. Verfahren gemäß einem der Ansprüche 11 bis 13, ferner umfassend die Schritte

a) Aufbringen mindestens einer weiteren flüssigen Probe enthaltend eine bekannte Konzentration des zu bestimmenden Analyten auf einen Aufnahmebereich einer Vorrichtung gemäß einem der vorhergehenden Ansprüche;

b) Optische Detektion des Reaktionsbereichs der Vorrichtung gemäß einem der vorhergehenden Ansprüche zu einem Zeitpunkt, an dem die Enzym-haltige und/oder Säure-haltige flüssige Probe von der Fluidleitung zu dem Reaktionsbereich transportiert wurde; und

c) Quantitative Bestimmung der Konzentration des Analyts in der Probe, bevorzugt über eine Bestimmung einer Anzahl und einem Volumens der Gasbläschen pro Zeiteinheit, besonders bevorzugt über den Zusammenhang, dass das Produkt aus Anzahl und Volumen der Gasbläschen pro Zeiteinheit direkt proportional ist zu der Konzentration des Analyts in der Probe.

**15.** Verwendung der Vorrichtung gemäß einem der Ansprüche 1 bis 10 zur

i) *in-vitro*-Diagnose einer Krankheit, wobei die flüssige Probe bevorzugt ausgewählt ist aus der Gruppe bestehend aus Blut, Urin, Sputum und Mischungen hiervon; und/oder

ii) Qualitätskontrolle von Lebensmitteln, bevorzugt zur Qualitätskontrolle einer Lebensmittelflüssigkeit, besonders bevorzugt zur Qualitätskontrolle von Wein, Fruchtsäften und Kombinationen hiervon; und/oder

iii) Untersuchung einer Qualität von Wasser, bevorzugt einer Qualität von Flusswasser, Seewasser, Meerwasser, Grundwasser, Trinkwasser, Abwasser und Kombinationen hiervon.

**Claims**

**1.** A device for detecting a specific analyte in a liquid sample via enzyme-catalyzed and/or acid-mediated conversion of the analyte to at least one gas, comprising

a) at least one fluid line;

b) at least one receiving region for receiving a liquid sample;

c)

i) at least one enzyme region comprising at least one specific enzyme capable of catalyzing the conversion of the analyte to be determined to at least one gas, said at least one enzyme region comprising biological cells and/or cell lysate, said biological cells and/or cell lysate comprising said at least one enzyme capable of catalyzing the conversion of the analyte to be determined to at least one gas; and/or

ii) at least one acidification region comprising at least one acid selected from the group consisting of acids solid at room temperature and normal pressure; and

d) at least one reaction region for forming gas bubbles, the reaction region comprising or consisting of a chamber fluidically connected to the at least one fluid line and having liquid-tight walls;

**characterized in that** the at least one fluid line is suitable for transporting the liquid sample from the receiving region via the at least one enzyme region and/or the at least one acidification region to the reaction region via capillary forces and/or at least one micropump in the fluid line.

**2.** The device according to claim 1, **characterized in that** the device

i) comprises a plurality of fluid lines, preferably a plurality of fluid lines that are suitable for transporting the liquid sample from the receiving region via at least one enzyme region and/or at least one acidification region to the reaction region via capillary forces and/or at least one micropump in the fluid line; and/or

ii) comprises a plurality of reaction regions, preferably a plurality of reaction regions for forming gas bubbles, the reaction regions each comprising or consisting of a chamber, each of which is fluidically connected to at least one fluid line and each of which has liquid-tight walls.

**3.** The device according to any one of the preceding claims, **characterized in that** the chamber comprises at least one wall, preferably at least two opposite walls, that

i) has a transparency for light of a wavelength in a range selected from the group consisting of IR range, visible range, UV range and combinations thereof, preferably having a transparency for light of a wavelength in the visible range; and/or

ii) has a tightness for liquids, gases and combinations thereof, preferably has a tightness for liquids.

4. The device according to any one of the preceding claims, **characterized in that** said at least one receiving region is suitable for receiving a liquid sample selected from the group consisting of aqueous solutions comprising or consisting of blood, urine, sputum, food, river water, lake water, sea water, ground water, drinking water, waste water and mixtures thereof.

5. The device according to any one of the preceding claims, **characterized in that** said at least one enzyme region

i) comprises at least one enzyme selected from the group consisting of urease, lactate oxidase, lactate dehydrogenase, catalase, pyruvate decarboxylase, thyreoperoxidase, and combinations thereof; and/or

ii) comprises at least one further enzyme, wherein the at least one further enzyme is preferably selected from the group consisting of catalase, pyruvate decarboxylase and combinations thereof;

iii) comprises at least one cofactor of an enzyme, preferably $NAD^+$; and/or

iv) is arranged between the receiving region for receiving a liquid sample and the reaction region for forming gas bubbles, or is arranged within the reaction region for forming gas bubbles; and/or

v) comprises the at least one enzyme and/or at least one cofactor of the at least one enzyme in dry form, preferably in lyophilized form, or in aqueous form, preferably as an aqueous solution, aqueous suspension or aqueous gel.

6. The device according to any one of the preceding claims, **characterized in that** the at least one fluid line comprises a membrane, which preferably

i) has a pore diameter of $\leq 20$ $\mu$m, preferably $\leq 6$ $\mu$m, particularly preferably $\leq 2$ $\mu$m, in particular $\leq 100$ nm, optionally $\leq 1$ nm; and/or

ii) is suitable for separating biological cells, preferably blood cells; and/or

iii) is arranged between the receiving region for receiving a liquid sample and the reaction region for forming gas bubbles, preferably between the receiving region for receiving a liquid sample and the enzyme region and/or the acidification region.

7. The device according to any one of the preceding claims, **characterized in that** the acidification region

i) comprises an acid selected from the group consisting of citric acid, ascorbic acid, malic acid, stearic acid, palmitic acid, myristic acid, lauric acid and mixtures thereof; and/or

ii) is arranged between the receiving region for receiving a liquid sample and the reaction region for forming gas bubbles or is arranged within the reaction region for forming gas bubbles.

8. The device according to any one of the preceding claims, **characterized in that** the device, preferably the at least one fluid line, comprises at least one oxidation region comprising at least one oxidizing agent, preferably at least one oxidizing agent and at least one acid, wherein the at least one oxidation region preferably

i) comprises an oxidizing agent selected from the group consisting of manganese dioxide, potassium permanganate, $NAD^+$ and mixtures thereof; and/or

ii) comprises an acid selected from the group consisting of acids solid at room temperature and normal pressure, particularly preferably citric acid, ascorbic acid, malic acid, stearic acid, palmitic acid, myristic acid, lauric acid and mixtures thereof; and/or

iii) is arranged between the receiving region for receiving a liquid sample and the reaction region for forming gas bubbles, particularly preferably between the enzyme region and the reaction region for forming gas bubbles, the at least one oxidation region overlapping in particular with the acidification region at least in regions or being identical therewith.

9. The device according to any one of the preceding claims, **characterized in that** the specific analyte

i) is selected from the group consisting of small organic molecule having a mass of < 500 Da, peptide, protein and mixtures thereof, preferably selected from the group consisting of hormone, metabolite having a mass of < 500 Da, carbohydrate, sterol, triglyceride, carboxylic acid, amide derivative of a carboxylic acid and mixtures thereof, particularly preferably selected from the group consisting of T3 hormone, T4 hormone, glucose, cholesterol, triglycerides of blood, lactic acid, urea and mixtures thereof; and/or

ii) is an analyte that is a marker for a condition selected from the group consisting of disease, water contamination, food contamination, and combinations thereof.

**10.** The device according to any one of the preceding claims, **characterized in that** the device is arranged in or at an optical detection device, preferably an optical microscope, the optical detection device being particularly preferably configured to optically detect the reaction region of the device and to perform a qualitative and/or quantitative determination of the concentration of the analyte in the sample, the detection device being particularly configured to

i) qualitatively determine a presence of the analyte in the sample when gas bubbles are formed in the reaction region; and/or
ii) quantitatively determine the concentration of the analyte in the sample via a number and volume of gas bubbles per unit time, in particular via the relationship that the product of the number and volume of gas bubbles per unit time is directly proportional to the concentration of the analyte in the sample.

**11.** A method for detecting a specific analyte in a liquid sample via enzyme-catalyzed and/or acid-mediated conversion of the analyte to at least one gas, comprising the steps of

i) applying a liquid sample possibly comprising the analyte to be determined to a receiving region of a device according to any one of the preceding claims;
ii) optically detecting the reaction region of the device according to any one of the preceding claims at a time when the enzyme-comprising and/or acid-comprising liquid sample has been transported from the fluid line to the reaction region; and
iii) determining that the specific analyte is present in the sample when gas bubbles are formed in the reaction region.

**12.** The method according to claim 11, **characterized in that** the optical detection is performed via an optical detection device selected from the group consisting of camera, microscope, photometer, refractometer and combinations thereof, preferably via a microscope.

**13.** The method according to any one of claims 11 or 12, **characterized in that** the method comprises a quantitative determination of the concentration of the analyte in the sample, preferably via a determination of the number and volume of gas bubbles per unit time, particularly preferably via the relationship that the product of the number and volume of gas bubbles per unit time is directly proportional to the concentration of the analyte in the sample.

**14.** The method according to any one of claims 11 to 13, further comprising the steps of

a) applying at least one further liquid sample comprising a known concentration of the analyte to be determined to a receiving region of a device according to any one of the preceding claims;
b) optically detecting the reaction region of the device according to any one of the preceding claims at a time when the enzyme-comprising and/or acid-comprising liquid sample has been transported from the fluid line to the reaction region; and
c) quantitatively determining the concentration of the analyte in the sample, preferably via a determination of a number and a volume of gas bubbles per unit time, particularly preferably via the relationship that the product of number and volume of gas bubbles per unit time is directly proportional to the concentration of the analyte in the sample.

**15.** A use of the device according to any one of claims 1 to 10 for

i) *in vitro* diagnosis of a disease, wherein the liquid sample is preferably selected from the group consisting of blood, urine, sputum and mixtures thereof; and/or
ii) quality control of food, preferably for quality control of a food liquid, particularly preferably for quality control of wine, fruit juices and combinations thereof; and/or
iii) examination of a quality of water, preferably a quality of river water, lake water, sea water, ground water, drinking water, waste water and combinations thereof.

**Revendications**

**1.** Dispositif de détection d'un analyte déterminé dans un échantillon liquide via une conversion enzymatique catalysée et/ou promue par un acide de l'analyte pour donner au moins un gaz, contenant

a) au moins une conduite de fluide ;

b) au moins une zone de réception permettant la réception d'un échantillon liquide ;

c) i) au moins une zone enzymatique qui contient au moins une enzyme déterminée, qui est appropriée pour catalyser la conversion de l'analyte à déterminer pour donner au moins un gaz, où l'au moins une zone enzymatique contient des cellules biologiques et/ou un lysat cellulaire, où les cellules biologiques et/ou le lysat cellulaire contiennent l'au moins une enzyme qui est appropriée pour catalyser la conversion de l'analyte à déterminer pour donner au moins un gaz ; et/ou ii) au moins une zone d'acidification qui contient au moins un acide qui est choisi dans le groupe constitué d'acides solides à la température ambiante et à la pression normale ; et

d) au moins une zone de réaction permettant la formation de bulles de gaz, où la zone de réaction contient une chambre ou en est constituée, qui est reliée de manière fluidique avec l'au moins une conduite de fluide et présente des parois étanches pour les liquides ;

**caractérisé en ce que** l'au moins une conduite de fluide est appropriée pour transporter l'échantillon liquide de la zone de réception vers la zone de réaction, via des forces capillaires et/ou au moins une micro pompe dans la conduite de fluide, en passant par l'au moins une zone enzymatique et/ou l'au moins une zone d'acidification.

2. Dispositif selon la revendication 1, **caractérisé en ce que** le dispositif

i) contient plusieurs conduites de fluide, de préférence, plusieurs conduites de fluide qui sont appropriées pour transporter l'échantillon liquide de la zone de réception vers la zone de réaction, via des forces capillaires et/ou au moins une micro pompe dans la conduite de fluide, en passant par l'au moins une zone enzymatique et/ou l'au moins une zone d'acidification ; et/ou

ii) contient plusieurs zones de réaction, de préférence, plusieurs zones de réaction pour la formation de bulles de gaz, où les zones de réaction contiennent respectivement une chambre ou en sont constituées, qui est reliée de manière fluidique avec l'au moins une conduite de fluide et présente des parois étanches pour les liquides.

3. Dispositif selon l'une des revendications précédentes, **caractérisé en ce que** la chambre contient au moins une paroi, de préférence, au moins deux parois situées l'une en face de l'autre, qui

i) présentent une transparence pour de la lumière dans une longueur d'ondes dans une plage choisie dans le groupe constitué du domaine IR, du domaine visible, du domaine UV et de combinaisons de ceux-ci, de préférence, une transparence pour de la lumière à des longueurs d'ondes dans le domaine visible ; et/ou

ii) présentent une étanchéité vis à vis des liquides, des gaz ou de combinaisons de ceux-ci, de préférence une étanchéité vis à vis des liquides.

4. Dispositif selon l'une des revendications précédentes, **caractérisé en ce que** l'au moins une zone de réception est appropriée pour la réception d'un échantillon liquide choisi dans le groupe constitué de solutions aqueuses ou constituées de sang, d'urine, d'expectorations, d'aliments, d'eau douce, d'eau de mer, d'eau souterraine, d'eau de boisson, d'eaux usées et de mélanges de ceux-ci.

5. Dispositif selon l'une des revendications précédentes, **caractérisé en ce que** l'au moins une zone enzymatique

i) contient au moins une enzyme choisie dans le groupe constitué de l'uréase, de la lactate oxydase, de la lactate déshydrogé-nase, de la catalase, de la pyruvate décarboxylase, de la thy-réoperoxydase et de combinaisons de celles-ci ; et/ou

ii) contient au moins une autre enzyme, où l'au moins une autre enzyme est choisie dans le groupe constitué de la catalase, de la pyruvate décarboxylase et de combinaisons de celles-ci ;

iii) contient au moins un cofacteur d'une enzyme, de préférence $NAD^+$ ; et/ou

iv) est disposée entre la zone de réception pour la réception d'un échantillon liquide et la zone de réaction pour la formation de bulles de gaz ou à l'intérieur de la zone de réaction pour la formation de bulles de gaz ; et/ou

v) contient l'au moins une enzyme et/ou l'au moins un cofacteur de l'au moins une enzyme sous une forme sèche, de préférence sous forme lyophilisée, ou sous forme aqueuse, de préférence sous forme de solution aqueuse, de suspension aqueuse ou de gel aqueux.

6. Dispositif selon l'une des revendications précédentes, **caractérisé en ce que** l'au moins une conduite de fluide contient une membrane qui présente de préférence

i) un diamètre de pore $\leq 20$ µm, de préférence $\leq 6$ µm, de manière particulièrement préférée $\leq 2$ µm, notamment $\leq 100$ nm, éventuellement $\leq 1$ nm ; et/ou

ii) est appropriée pour la séparation de cellules biologiques, de préférence pour la séparation de cellules sanguines ; et/ou

iii) est disposée entre la zone de réception pour la réception d'un échantillon liquide et la zone de réaction pour la formation de bulles de gaz, de préférence entre la zone de réception pour la réception d'un échantillon liquide et la zone enzymatique, et/ou la zone d'acidification.

7. Dispositif selon l'une des revendications précédentes, **caractérisé en ce que** la zone d'acidification

i) contient un acide, qui est choisi dans le groupe constitué de l'acide citrique, de l'acide ascorbique, de l'acide malique, de l'acide stéarique, de l'acide palmitique, de l'acide myristique, de l'acide Laurique et de mélanges de ceux-ci ; et/ou qui

ii) est disposé entre la zone de réception pour la réception d'un échantillon liquide et la zone de réaction pour la formation de bulles de gaz ou à l'intérieur de la zone de réaction pour la formation de bulles de gaz.

8. Dispositif selon l'une des revendications précédentes, **caractérisé en ce que** le dispositif contient l'au moins une conduite de fluide, au moins une zone d'oxydation, qui contient au moins un agent d'oxydation, de préférence, au moins un agent d'oxydation et au moins un acide, où l'au moins une zone d'oxydation contient de préférence

i) un agent d'oxydation qui est choisi dans le groupe constitué du dioxyde de manganèse, du permanganate de potassium, de NAD$^+$ et de mélanges de ceux-ci ; et/ou qui contient

ii) un acide qui est choisi dans le groupe constitué d'acides solides à la température ambiante et à la pression normale, de manière particulièrement préférée, de l'acide citrique, de l'acide ascorbique, de l'acide malique, de l'acide stéarique, de l'acide palmitique, de l'acide myristique, de l'acide Laurique et de mélanges de ceux-ci ; et/ou

iii) est disposée entre la zone de réception pour la réception d'un échantillon liquide et la zone de réaction pour la formation de bulles de gaz, de manière particulièrement préférée entre la zone enzymatique et la zone de réaction pour la formation de bulles de gaz, où l'au moins une zone d'oxydation se superpose par endroits en particulier avec la zone d'acidification ou est identique à celle-ci.

9. Dispositif selon l'une des revendications précédentes, **caractérisé en ce que** l'analyte déterminé

i) est choisi dans le groupe constitué de petites molécules organiques pourvues d'une masse < 500 Da, de peptides, de protéines et de mélanges de ceux-ci, choisies de préférence dans le groupe constitué d'hormones, de métabolites avec une masse < 500 Da, d'hydrates de carbone, de stérols, d'acides carboniques de triglycérides, de dérivés d'amides d'un acide carboxylique et de mélanges de ceux-ci, de manière particulièrement préférée choisis dans le groupe constitué de l'hormone T3, de l'hormone T4, du glucose, du cholestérol, de triglycérides de l'acide lactique de sang, de l'urée et de mélanges de ceux-ci ; et/ou

ii) est un analyte qui est un marqueur pour un état qui est choisi dans le groupe constitué d'une maladie, d'une pollution de l'eau, d'une pollution de produits alimentaires et de combinaisons de celles-ci.

10. Dispositif selon l'une des revendications précédentes, **caractérisé en ce que** le dispositif est disposé sur un appareil de détection optique, de préférence, un microscope optique, où l'appareil de détection optique est conçu de manière particulièrement préférée pour détecter optiquement la zone de réaction du dispositif et effectuer une détermination qualitative et/ou quantitative de la concentration de l'analyte dans l'échantillon, où l'appareil de détection est en particulier conçu

i) pour déterminer qualitativement la présence de l'analyte dans l'échantillon lorsqu'il arrive dans la zone de réaction pour la formation de bulles de gaz ; et/ou

ii) pour déterminer quantitativement la concentration de l'analyte dans l'échantillon par le biais d'un nombre et d'un volume de bulles de gaz par unité de temps, en particulier par la relation qui fait que le produit est directement proportionnel au nombre et au volume des bulles de gaz par unité de temps et à la concentration de l'analyte dans l'échantillon.

11. Procédé de détection d'un analyte déterminé dans un échantillon liquide via une conversion de l'analyte catalysée par une enzyme et/ou promue par un acide pour donner au moins un gaz, comprenant les étapes

i) d'application d'un échantillon liquide qui contient peut-être l'analyte à déterminer, sur une zone de réception d'un dispositif selon l'une des revendications précédentes ;

ii) de détection optique de la zone de réaction du dispositif selon l'une des revendications précédentes à un moment où l'échantillon liquide contenant l'enzyme et/ou l'acide a été transporté vers la zone de réaction par la conduite de fluide ; et

iii) de détermination que l'analyte déterminé est présent dans l'échantillon lorsqu'il se produit la formation de bulles de gaz dans la zone de réaction.

12. Procédé selon la revendication 11, **caractérisé en ce que** la détection optique a lieu par un appareil de détection optique choisi dans le groupe constitué d'une caméra, d'un microscope, d'un photomètre, d'un réfractomètre et de combinaisons de ceux-ci, de préférence par un microscope.

13. Procédé selon l'une des revendications 11 ou 12, **caractérisé en ce que** le procédé comprend une détermination quantitative de la concentration de l'analyte dans l'échantillon, de préférence, par le biais d'une détermination du nombre et du volume des bulles de gaz par unité de temps, de manière particulièrement préférée par la relation qui fait que le produit est directement proportionnel au nombre et au volume des bulles de gaz par unité de temps et à la concentration de l'analyte dans l'échantillon.

14. Procédé selon l'une des revendications 11 à 13, comprenant en outre les étapes

a) d'application d'au moins un nouvel échantillon liquide contenant une concentration connue de l'analyte à déterminer sur une zone de réception d'un dispositif selon l'une des revendications précédentes ;

b) de détection optique de la zone de réaction du dispositif selon l'une des revendications précédentes à un moment où l'échantillon liquide contenant l'enzyme et/ou l'acide a été transporté vers la zone de réaction par la conduite de fluide ; et

c) la détermination quantitative de la concentration de l'analyte dans l'échantillon, de préférence par le biais d'une détermination d'un nombre et d'un volume de bulles de gaz par unité de temps, de manière particulièrement préférée par la relation que le produit est directement proportionnel au nombre et au volume des bulles de gaz par unité de temps et à la concentration de l'analyte dans l'échantillon.

15. Utilisation du dispositif selon l'une des revendications 1 à 10 pour

i) le diagnostic *in-vitro* d'une maladie, où l'échantillon liquide est de préférence choisi dans le groupe constitué de sang, d'urine, d'expectorations et de mélanges de ceux-ci ; et/ou

ii) le contrôle qualité de produits alimentaires, de préférence, le contrôle qualité d'un liquide de produit alimentaire, de manière particulièrement préférée pour le contrôle qualité de vin, de jus de fruits et de combinaisons de ceux-ci ; et/ou

iii) l'analyse d'une qualité de l'eau, de préférence, d'une qualité d'eau douce, d'eau d'un lac, d'eau de mer, d'eau souterraine, d'eau de boisson, d'eaux usées et de combinaisons de celles-ci.

# Figur 1

| Analyt | Enzym und/oder Säure (optional mit Oxidationsmittel) | Gas | Chemische Reaktion |
|---|---|---|---|
| **Carbonate** | Säure | Kohlenstoffdioxid | Säure-Base-Reaktion<br>$CO_3^{2-}$ + 2 HCl $\leftrightarrow$ 2 Cl$^-$ + $H_2O$ + **$CO_2$** |
| **Aldehyde** | schwachsaure Kaliumpermanganat-lösung | Kohlenstoffdioxid | Oxidation<br>5 $C_3H_6O_3$ + $MnO_4^-$ + 6 $H_3O^+$ $\rightarrow$ 5 $C_2H_4O$ + 5 **$CO_2$** + 2 $Mn^{2+}$ + 14 $H_2O$ |
| **Harnstoff** | Urease | Kohlenstoffdioxid | Enzymatische Reaktion<br>$CH_4N_2O$ + 2 $H_2O$ + Urease $\rightarrow$ 2 $NH_3$ + **$CO_2$** |
| **Milchsäure (Laktat)** | ▪ Laktatoxidase<br>▪ schwachsaure Kaliumpermanganat-lösung | Sauerstoff | Enzymatische Reaktion<br>▪ $C_3H_6O_3$ + $O_2$ + Laktatoxidase $\rightarrow$ $C_3H_4O_3$ + $H_2O_2$<br>Oxidation<br>▪ 2 $MnO_4^-$ + $H_2O_2$ + 6 $H^+$ $\rightarrow$ 2 $Mn^{2+}$ + 8 $H_2O$ + 5 **$O_2$** |
| | ▪ Laktatoxidase<br>▪ Katalase | Sauerstoff | Enzymatische Reaktion<br>▪ $C_3H_6O_3$ + $O_2$ + Laktatoxidase $\rightarrow$ $C_3H_4O_3$ + $H_2O_2$<br>Enzymatische Reaktion<br>▪ 2 $H_2O_2$ + Katalase $\rightarrow$ **$O_2$** + 2 $H_2O$ |
| | ▪ Laktatdehydrogenase<br>▪ NAD$^+$<br>▪ Pyruvatdecarboxylase | Kohlenstoffdioxid | Enzymatische Reaktion<br>▪ $C_3H_6O_3$ + NAD$^+$ + Laktatdehydrogenase $\rightarrow$ $C_3H_4O_3$ + NADH + $H^+$<br>Enzymatische Reaktion<br>▪ $C_3H_4O_3$ + Pyruvatdecarboxylase $\rightarrow$ $C_2H_4O$ + **$CO_2$** |
| **Glucose** | Pyruvatdecarboxylase | Kohlenstoffdioxid | Enzymatische Reaktion<br>▪ $C_6H_{12}O_6$ $\rightarrow$ 2 $C_2H_5OH$ + 2 **$CO_2$** |

Figur 2

Figur 3

Figur 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2009125227 A